# EUROPEAN PATENT SPECIFICATION

(11) **EP 3 634 255 B1**
(45) Date of publication and mention of the grant of the patent: **12.08.2020**
(21) Application number: 18734730.7
(22) Date of filing: 08.06.2018
(51) Int. Cl.: A61B 17/04, A61F 2/24, A61F 2/08, A61B 17/00, A61B 17/064

(54) **TISSUE ANCHOR WITH TETHER STOP**
GEWEBEANKER MIT HALTEGURTANSCHLAG
ANCRE DE TISSU AVEC BUTÉE D'ATTACHE

(30) Priority: 08.06.2017 US 201762516894 P
(43) Date of publication of application: 15.04.2020
(73) Proprietor: 4Tech Inc., Waltham, MA 02452-8496 (US)
(72) Inventor: GRIFFIN, Patrick, Castlegar Galway (IE)
(74) Representative: Evens, Paul Jonathan
(86) International application number: PCT/US2018/036609
(87) International publication number: WO 2018/227048

(56) References cited:
- EP-A1- 3 068 311
- WO-A2-2016/189391
- US-A1- 2004 260 317
- US-A1- 2008 228 267
- US-A1- 2011 029 071
- US-A1- 2011 106 245
- US-A1- 2015 018 876

## Description

### CROSS-REFERENCE TO RELATED APPLICATIONS

The present application claims the benefit of US Provisional Application 62/516,894, filed June 8, 2017, which is assigned to the assignee of the present application

### FIELD OF THE APPLICATION

The present invention relates generally to tissue anchors, and specifically to tissue anchors for implantation at cardiac sites.

### BACKGROUND OF THE APPLICATION

Tissue anchors are used for anchoring elements, such as pacemaker electrode leads or sutures, to tissue, such as bone or soft tissue. PCT Publication WO 2016/087934 to Gilmore et al. describes a tissue anchor that includes a shaft, a tissue-coupling element, and a flexible elongate tension member. The tissue-coupling element includes a wire, which is shaped as an open loop coil having, in some applications, more than one coil revolution when the tissue anchor is unconstrained, i.e., expanded from a linear state to a coiled state. The tension member includes a distal portion, that is fixed to a site on the open loop coil, a proximal portion, which has a longitudinal segment that runs alongside at least a portion of the shaft, and a crossing portion, which (i) is disposed between the distal and the proximal portions along the tension member, and (ii) crosses at least a portion of the open loop when the tissue anchor is expanded. The tissue anchor is configured to allow relative axial motion between the at least a portion of the shaft and the longitudinal segment of the proximal portion of the tension member when the tissue anchor is expanded. For some applications, a head of the tissue anchor is shaped so as to define a passage in which the proximal portion of the flexible elongate tension member is slidably disposed. The flexible elongate tension member comprises a locking stopper, which is axially fixed to the proximal or the crossing portion of the flexible elongate tension member. The locking stopper and the passage are sized and shaped such that the size and shape of the passage prevent proximal movement of the locking stopper past the passage. The locking stopper limits the total load that can be applied to the open loop by the flexible elongate tension member, thereby reducing excessive, unnecessary strain on the open loop. Additional load (tension) that is applied by the flexible elongate tension member pulls on the entire anchor, and does not further increase the load applied across the open loop.

EP 3068311 relates to off-center tissue anchors, and discloses an expandable tissue anchor according to the pre-characterizing portion of appended claim 1.

US 2011/0106245 relates to repair of an atrioventricular valve, and discloses apparatus comprising a tissue anchor.

### SUMMARY OF THE APPLICATION

In accordance with the present invention, there is provided an expandable tissue anchor as defined in appended independent claim 1. Embodiments of the present invention are defined in appended claims which depend upon independent claim 1. Methods of using the expandable tissue anchor are presented as a way to understand the invention and do not form part of the invention.

Embodiments of the present invention provide expandable tissue anchors that are deliverable to a cardiac chamber in an unexpanded generally elongate configuration within a deployment tool. The expandable tissue anchors are configured to be anchored to a cardiac tissue wall at a target site such that a tensile force can be applied to the expandable tissue anchors and thus to the cardiac tissue wall, once the expandable tissue anchors are deployed, so as to move the cardiac tissue wall at the target site relative to adjacent cardiac tissue. For some applications, such motion alters the geometry of a cardiac valve, such as the tricuspid valve or the mitral valve.

In some applications of the present invention, an expandable tissue anchor comprises an elongate tissue-coupling portion, which is configured to expand on a second side of the cardiac tissue wall. The expandable tissue anchor further comprises a flexible elongate tension member coupled to a portion of the tissue-coupling portion of the expandable tissue anchor, such that a tensile force can be applied to the tissue-coupling portion after it has been expanded. The expandable tissue anchor further comprises a sleeve that encloses a portion of the flexible elongate tension member between (a) the tissue-coupling portion of the expandable tissue anchor and (b) a distal opening of the passage. The sleeve and a passage through the expandable tissue anchor are sized and shaped such that the size and shape of the passage prevent proximal movement of the sleeve past the passage, thereby limiting compression and deformation of the expanded tissue-coupling portion by the flexible elongate tension member.

There is therefore provided, in accordance with an application of the present invention, an expandable tissue anchor configured to be delivered to a cardiac chamber using a deployment tool, and to be anchored to a cardiac tissue wall at a target site such that a tensile force can be applied to the expandable tissue anchor and thus to the cardiac tissue wall, once the expandable tissue anchor is deployed, so as to move the cardiac tissue wall at the target site relative to adjacent cardiac tissue, the expandable tissue anchor including:
an elongate tissue-coupling portion configured to be delivered in an unexpanded generally elongate configuration through the cardiac tissue wall from a first side of the cardiac tissue wall to a second side of the cardiac tissue wall, the tissue-coupling portion further configured to expand on the second side of the cardiac tissue wall;
a flexible elongate tension member, which is coupled to a portion of the tissue-coupling portion of the expandable tissue anchor such that the tensile force can be applied to the tissue-coupling portion after it has been expanded, wherein a portion of the flexible elongate tension member is slidably disposed through a passage defined by the expandable tissue anchor;
an anchor head that supports the tissue-coupling portion at a proximal end of the tissue-coupling portion; and
a sleeve that encloses a portion of the flexible elongate tension member between (a) the tissue-coupling portion of the expandable tissue anchor and (b) a distal end of the passage, with the sleeve and the passage being sized and shaped such that the size and shape of the passage prevent proximal movement of the sleeve past the passage upon application of the tensile force to the flexible elongate tension member, thereby limiting compression and deformation of the expanded tissue-coupling portion by the flexible elongate tension member,
characterized in that: the sleeve is axially slidable along the flexible elongate tension member, with the sleeve and the passage limiting movement of the tissue-coupling portion toward a distal end of the anchor head upon application of the tensile force to the flexible elongate tension member, because (a) the sleeve has sufficient axial stiffness and too large an outer diameter to pass through the passage, and (b) a distal end of the sleeve contacts the tissue-coupling portion near a junction between the flexible elongate tension member and the tissue-coupling portion; and
the sleeve is configured such that the expanded tissue-coupling portion and the sleeve can be drawn tightly against the second side of the cardiac tissue wall at the target site when the tensile force is applied to the tissue-coupling portion after it has been expanded.

For some applications, the expandable tissue anchor is configured such that when the cardiac tissue wall is a myocardial tissue wall, the tissue-coupling portion of the expandable tissue anchor can be advanced into the pericardial cavity between visceral pericardium and parietal pericardium, generally alongside and against the parietal pericardium, without penetrating the parietal pericardium.

For some applications, the anchor head is shaped so as to define the passage.

For some applications, a lateral surface of the anchor head is shaped so as to define at least a portion of the distal opening of the passage.

For some applications, the anchor head includes a collar, which is shaped so as to define the distal opening of the passage.

For some applications, a distal end of the collar is shaped so as to define the distal opening of the passage.

For some applications, a lateral surface of the collar is shaped so as to define at least a portion of the distal opening of the passage.

For some applications, the tissue-coupling portion of the expandable tissue anchor, once expanded on the second side of the cardiac tissue wall, assumes a shape generally orthogonal to the anchor head.

There is further provided, in accordance with an application of the present invention, an anchor system including the expandable tissue anchor, wherein the anchor system further includes a tether affixed to the flexible elongate tension member such that the tensile force can be applied to the expandable tissue anchor via the tether and the flexible elongate tension member.

There is still further provided a method for moving a cardiac tissue wall at a target site relative to adjacent cardiac tissue, the method including:
delivering, to a cardiac chamber, an expandable tissue anchor in an unexpanded generally elongate configuration within a deployment tool, the expandable tissue anchor including (a) an elongate tissue-coupling portion, (b) a flexible elongate tension member coupled to a portion of the tissue-coupling portion, and (c) a sleeve that encloses a portion of the flexible elongate tension member between (i) the tissue-coupling portion of the expandable tissue anchor and (ii) a distal opening of a passage defined by the expandable tissue anchor, wherein a portion of the flexible elongate tension member is slidably disposed through the passage defined by the expandable tissue anchor;
delivering the tissue-coupling portion in an unexpanded generally elongate configuration through the cardiac tissue wall from a first side of the wall to a second side of the wall, such that the tissue-coupling portion expands on the second side of the cardiac tissue wall, thereby anchoring the expandable tissue anchor to the cardiac tissue wall at the target site;
partially compressing the expanded tissue-coupling portion by applying a tensile force to the flexible elongate tension member, until the passage prevents proximal movement of the sleeve past the passage, thereby limiting compression and deformation of the expanded tissue-coupling portion by the flexible elongate tension member; and
after the passage prevents proximal movement of the sleeve past the passage, applying, to the flexible elongate tension member, additional tensile force that does not further compress or deform the expanded tissue-coupling portion, and thus is applied to the cardiac tissue wall, so as to move the cardiac tissue wall at the target site relative to the adjacent cardiac tissue.

The expandable tissue anchor further includes an anchor head that supports the tissue-coupling portion at a proximal end of the tissue-coupling portion.

For some applications, the anchor head is shaped so as to define the passage.

For some applications, a lateral surface of the anchor head is shaped so as to define at least a portion of the distal opening of the passage.

For some applications, the anchor head includes a collar, which is shaped so as to define the distal opening of the passage.

For some applications, a distal end of the collar is shaped so as to define the distal opening of the passage.

For some applications, a lateral surface of the collar is shaped so as to define at least a portion of the distal opening of the passage.

For some applications, the tissue-coupling portion and the expandable tissue anchor, once expanded on the second side of the cardiac tissue wall, includes a configuration generally orthogonal to the anchor head.

The sleeve is axially slidable along the flexible elongate tension member.

Applying the tensile force to the flexible elongate tension member includes tightly drawing the expanded tissue-coupling portion and the sleeve against the second side of the cardiac tissue wall at the target site.

For some applications, the cardiac tissue is a myocardial tissue wall, and delivering the tissue-coupling portion in the unexpanded generally elongate configuration through the cardiac tissue wall includes delivering the tissue-coupling portion through the cardiac tissue wall into the pericardial cavity between visceral pericardium and parietal pericardium, generally alongside and against the parietal pericardium, without penetrating the parietal pericardium.

For some applications, applying the tensile force to the flexible elongate tension member includes applying the tensile force to a tether affixed to the flexible elongate tension member.

The present invention will be more fully understood from the following detailed description of embodiments thereof, taken together with the drawings, in which:

### BRIEF DESCRIPTION OF THE DRAWINGS

Fig. 1 is a schematic illustration of an expandable tissue anchor that is configured to be anchored to a cardiac tissue wall at a target site, in accordance with an application of the present invention;
Figs. 2A-C are schematic illustrations of a method of deploying the expandable tissue anchor of Fig. 1 through a myocardial tissue wall;
Fig. 3 is a schematic illustration of another expandable tissue anchor, in accordance with an application of the present invention; and
Fig. 4 is a schematic illustration of yet another expandable tissue anchor, in accordance with an application of the present invention.

### DETAILED DESCRIPTION OF APPLICATIONS

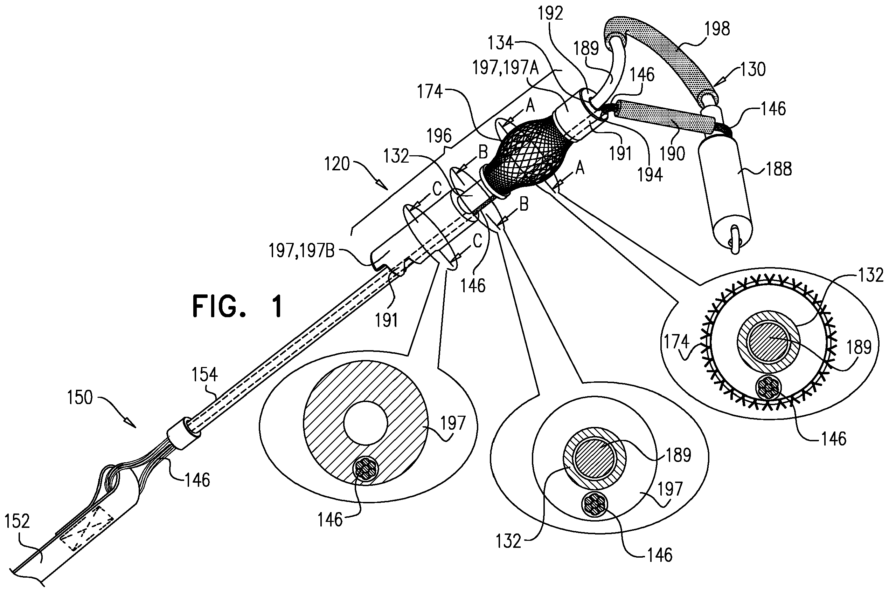

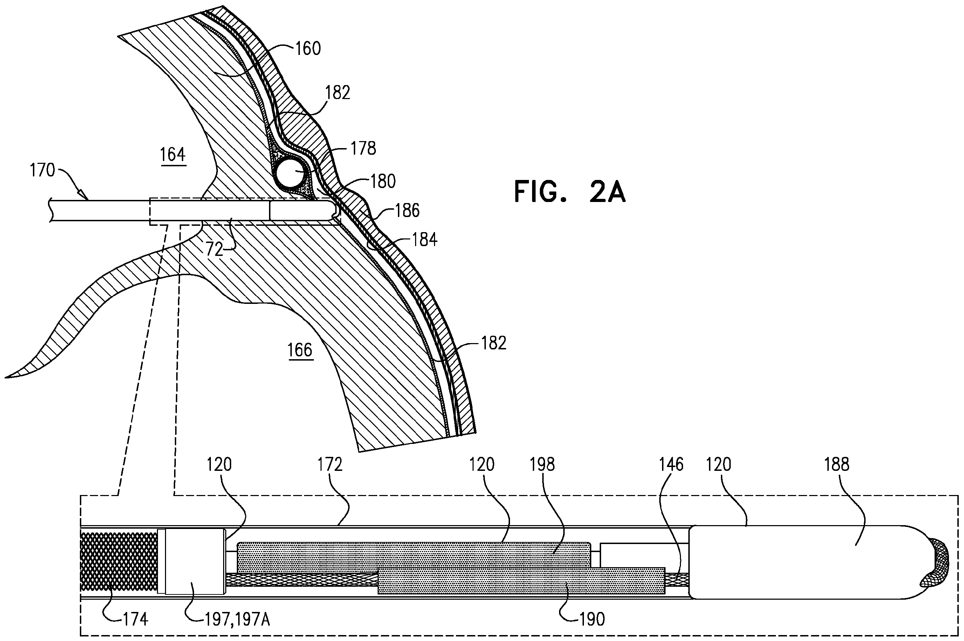

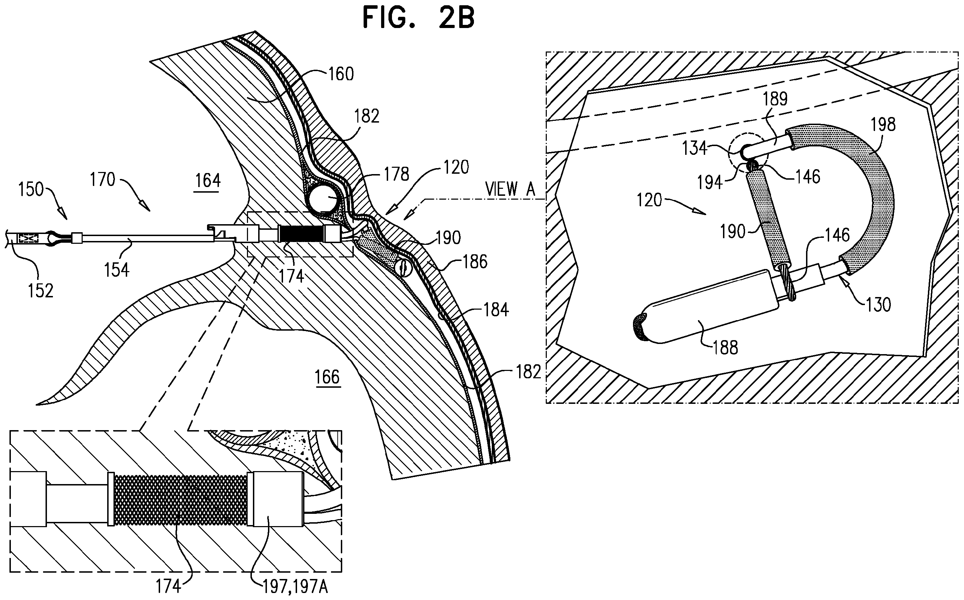

Fig. 1 is a schematic illustration of an expandable tissue anchor 120 that is configured to be anchored to a cardiac tissue wall at a target site such that a tensile force can be applied to expandable tissue anchor 120 and thus to the cardiac tissue wall, so as to move the cardiac tissue wall at the target site relative to adjacent cardiac tissue, in accordance with an application of the present invention. Expandable tissue anchor 120 comprises an elongate tissue-coupling portion 130, which optionally is supported by an anchor head 196 at a proximal end 134 of tissue-coupling portion 130. Tissue-coupling portion 130 is configured to be delivered in an unexpanded generally elongate configuration, such as described hereinbelow with reference to Fig. 2A, through the cardiac tissue wall from a first side of the wall to a second side of the wall, such as described hereinbelow with reference to Figs. 2A-C. Tissue-coupling portion 130 is further configured, upon deployment, to expand on the second side of the cardiac tissue wall, such as described hereinbelow with reference to Fig. 2B. Figs. 1 and 2B show tissue-coupling portion 130 expanded. For some applications, expanded tissue-coupling portion 130 has less than one turn, as shown in the figures, while for other applications, expanded tissue-coupling portion 130 has one turn (configuration not shown) or more than one turn (configuration not shown, but, for example, may be as shown in Figs. 5B-D, 6A-B, 7A-B, 9A-G, and/or 91 of above-mentioned PCT Publication WO 2016/087934).

Expandable tissue anchor 120 further comprises a flexible elongate tension member 146 coupled to a portion of tissue-coupling portion 130 of expandable tissue anchor 120. Through flexible elongate tension member 146, or components equivalent thereto, the tensile force can be applied to tissue-coupling portion 130 after it has been expanded. When applied *in vivo,* the tensile force may have the benefit of bringing the anchor close to the tissue wall to which it is applied. For some applications, an anchor system 150 is provided that comprises expandable tissue anchor 120 and a tether 152 affixed to flexible elongate tension member 146 such that the tensile force can be applied to expandable tissue anchor 120 via tether 152 and flexible elongate tension member 146. Optionally, expandable tissue anchor 120 further comprises a tube 154 that surrounds a proximal portion of flexible elongate tension member 146. For some applications, anchor system 150 further comprises a second tissue anchor, separate and distinct from expandable tissue anchor 120, such as is shown in above-mentioned PCT Publication WO 2016/087934. For some applications, the second tissue anchor, and additional anchors if so desired, is couplable or coupled to expandable tissue anchor 120 by one or more tethers that include tether 152.

Expandable tissue anchor 120 further comprises a sleeve 190 that encloses a portion of flexible elongate tension member 146 between (a) tissue-coupling portion 130 of expandable tissue anchor 120 and (b) a distal opening 194 of a passage 191 through expandable tissue anchor 120, such that expanded tissue-coupling portion 130 (and, optionally, sleeve 190) can be drawn tightly against the second side of the cardiac tissue wall at the target site when the tensile force is applied to tissue-coupling portion 130.

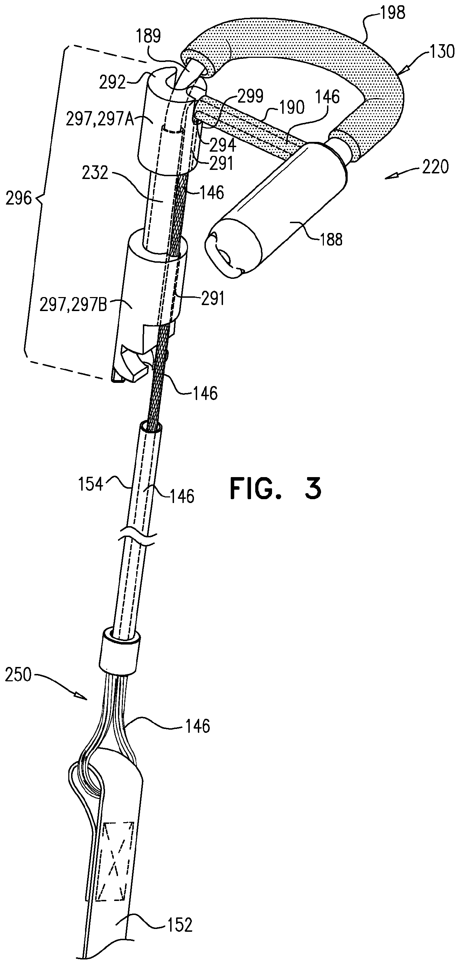

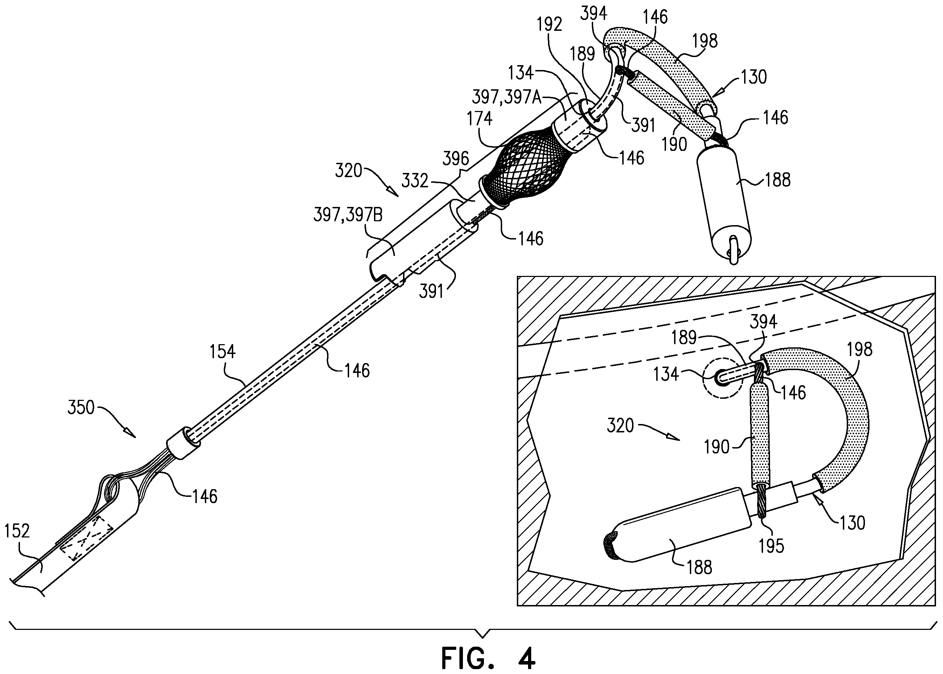

Distal opening 194 of passage 191 is typically located near (e.g., at) a distal end 192 of anchor head 196. A portion of flexible elongate tension member 146 is slidably disposed through passage 191. For some applications, passage 191 is defined by anchor head 196 (as shown). For example, distal opening 194 may be defined by a tubular anchor shaft 132 that anchor head 196 comprises; anchor head 196 may optionally implement techniques described in above-mentioned PCT Publication WO 2016/087934. For some applications, in addition to or instead of tubular anchor shaft 132, anchor head 196 comprises one or more collars 197, such as distal and proximal collars 197A and 197B, as shown, or exactly one collar 197 (configuration not shown). For some of these applications, distal opening 194 is defined by a distal end of distal collar 197A (as shown in Figs. 1 and 2A-C) or a distal end of the exactly one collar 197 (configuration not shown). For other applications, distal opening 194 is defined by a lateral surface of the distal collar, such as described hereinbelow with reference to Fig. 3. For some applications, passage 191 includes more than one passage 191, as shown. For some applications, passage 191 is alternatively or additionally defined by another portion of expandable tissue anchor 120, such as both tissue-coupling portion 130 of expandable tissue anchor 120 and anchor head 196 of expandable tissue anchor 120, such as shown in Fig. 4, described hereinbelow. Passage 191 is typically a channel, but may also be a groove (e.g., a U-shaped groove).

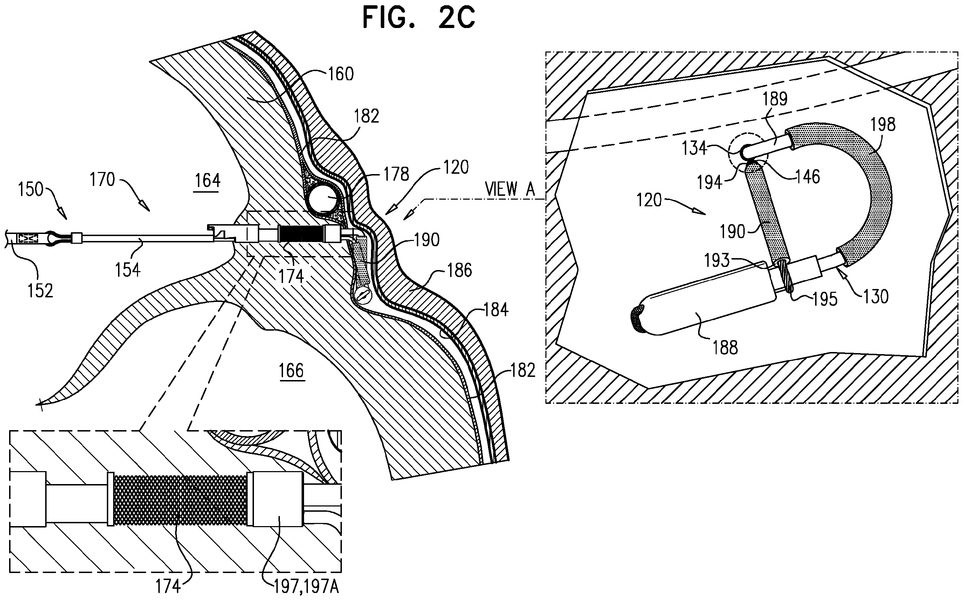

Sleeve 190 and passage 191 are sized and shaped such that the size and shape of passage 191 prevent proximal movement of sleeve 190 past passage 191 (e.g., prevent proximal movement of sleeve 190 into distal opening 194). Sleeve 190 and passage 191 thus limit movement of tissue-coupling portion 130 toward distal end 192 of anchor head 196 upon application of the tensile force to flexible elongate tension member 146. This is the case because sleeve 190 has sufficient axial stiffness and too large an outer diameter to pass through passage 191 (e.g., including distal opening 194), and a distal (far) end 193 of sleeve 190 contacts tissue-coupling portion 130 near a junction 195 between flexible elongate tension member 146 and tissue-coupling portion 130, such as shown in Fig. 2C. Sleeve 190, because of its axial stiffness, thus limits compression and deformation of expanded tissue-coupling portion 130 by flexible elongate tension member 146. A portion of the load (tension) applied by flexible elongate tension member 146 brings sleeve 190 into contact with expandable tissue anchor 120 near or at distal opening 194. Additional load (tension) applied by flexible elongate tension member 146 (a) increases the total load on expanded tissue-coupling portion 130 (without further compressing or deforming expanded tissue-coupling portion 130), (b) optionally is diverted to elastic or plastic deformation of sleeve 190, and (c) pulls on the entire expandable tissue anchor 120.

Attachment of sleeve 190 to flexible elongate tension member 146 is relatively simple during manufacture of expandable tissue anchor 120, as sleeve 190 need not be, and indeed typically is not, axially fixed to flexible elongate tension member 146. Even though sleeve 190 is typically axially slidable along flexible elongate tension member 146, the sleeve nevertheless serves its compression-limiting function, as described hereinabove.

In addition, sleeve 190 may generally increase the contact area between flexible elongate tension member 146 and the second site of the cardiac tissue wall. As a result, sleeve 190 may generally reduce the likelihood of flexible elongate tension member 146 cutting the cardiac tissue wall, particularly if the tissue is diseased.

For some applications, sleeve 190 has an outer diameter of at least 0.5 mm, no more than 1 mm, and/or between 0.5 and 1 mm, such as 0.75 mm. For some applications, sleeve 190 has a length of at least 2 mm, no more than 6 mm, and/or between 2 and 6 mm, such as between 3 and 5 mm, e.g., 4 mm. For some applications, sleeve 190 comprises PET, PEEK, a closely wound metal coil spring, porous ePTFE, or woven PET fiber.

For some applications, tissue-coupling portion 130, once expanded on the second side of the cardiac tissue wall, such as described hereinbelow with reference to Fig. 2B, assumes a shape generally orthogonal to anchor head 196, as shown in Fig. 1, although it need not be orthogonal.

For some applications, as shown in Fig. 1, anchor head 196 further comprises a sealing element 174, which is sized and shaped to be inserted with anchor head 196 into an incision through the cardiac tissue wall. Sealing element 174, along with at least a portion of anchor head 196, remains in the incision upon completion of the implantation of expandable tissue anchor 120. Sealing element 174 promotes hemostasis to provide sealing of the incision. For some applications, sealing element 174 comprises a mesh, which may comprise Nitinol, covered with a membrane. The membrane may comprise a bioabsorbable polymer, which breaks down after healing and hemostasis occur.

Reference is now made to Figs. 2A-C, which are schematic illustrations of a method of deploying expandable tissue anchor 120 through a myocardial tissue wall 160. Although in Figs. 2A-C expandable tissue anchor 120 is shown deployed through a myocardial tissue wall, expandable tissue anchor 120 may also be deployed through other cardiac tissue walls, such as the interatrial septum, either at or not at the fossa ovalis, or through other non-cardiac tissue walls. Indeed, the tissue anchors described herein may be deployed in any number of bodily locations where it is desired to anchor into or behind tissue for purposes of moving such tissue relative to adjacent tissue.

As shown in Fig. 2A, expandable tissue anchor 120 is delivered to a target site, such as a cardiac chamber, in an unexpanded generally elongate configuration within a deployment tool 170, which may comprise a hollow needle 172. The cardiac chamber may be a right atrium 164 (as shown), a right ventricle 166 (configuration not shown), a left atrium (configuration not shown), or a left ventricle (configuration not shown). In one application, hollow needle 172 is used to puncture through a first side of a myocardial tissue wall 160 and visceral pericardium 182 (which is part of the epicardium), avoiding vasculature such as the right coronary artery (RCA) 178. For some applications, deployment tool 170 is then further directed into the pericardial cavity 180 between visceral pericardium 182 and parietal pericardium 184, carefully avoiding puncturing parietal pericardium 184 and fibrous pericardium 186.

As shown in Fig. 2B, tissue-coupling portion 130 expands on the second side of myocardial tissue wall 160, thereby anchoring expandable tissue anchor 120 to myocardial tissue wall 160.

As shown in Fig. 2C, once expandable tissue anchor 120 has been anchored to myocardial tissue wall 160 at the target site, expanded tissue-coupling portion 130 (and, optionally, sleeve 190) is tightly drawn against the second side of myocardial tissue wall 160 at the target site by applying a tensile force, using tether 152, to tissue-coupling portion 130 and thus to myocardial tissue wall 160. Application of the tensile force partially compresses expanded tissue-coupling portion 130, until passage 191 prevents proximal movement of sleeve 190 past passage 191, thereby limiting movement of tissue-coupling portion 130 toward distal end 192 of anchor head 196 and thus further compression and deformation of expanded tissue-coupling portion 130. Typically, after passage 191 prevents the proximal movement of the sleeve 190 past the passage, addition tensile force is applied to flexible elongate tension member 146 that does not further compress or deform the expanded tissue-coupling portion 130. As a result, the additional tensile force is applied to myocardial tissue wall 160 and myocardial tissue wall 160 at the target site is moved relative to adjacent cardiac tissue. For some applications, such motion can have the benefit of altering the geometry of a nearby cardiac valve, such as the tricuspid valve or the mitral valve.

For some applications, after application of the tensile force, all or a portion of sleeve 190 rests against the second side of myocardial tissue wall 160, generally helping prevent possible inadvertent cutting of myocardial tissue wall 160 by flexible elongate tension member 146. For some applications, such as shown in Fig. 2B, tissue-coupling portion 130 (and sleeve 190) is delivered through myocardial tissue wall 160, into pericardial cavity 180, generally alongside and against parietal pericardium 184, without penetrating the parietal pericardium 184. For some applications, a second tissue anchor is implanted, separate and distinct from expandable tissue anchor 120.

Reference is made to Figs. 1 and 2A-C. For some applications, tissue-coupling portion 130 comprises a tip 188, which is fixed to a distal end of a wire 189 of tissue-coupling portion 130. Tip 188, at a widest longitudinal site along tip 188, has a greatest outer cross-sectional area that equals at least 150% (e.g., at least 200%, or at least 300%) of an average cross-sectional area of wire 189. (The cross-sectional area of tip 188 is measured perpendicular to a central longitudinal axis of tip 188. Similarly, the cross-sectional area of wire 189 is measured perpendicular to a central longitudinal axis of the wire, and is not a cross-sectional area of tissue-coupling portion 130.) Tip 188 temporarily serves as an atraumatic distal end of hollow needle 172 of deployment tool 170 when the tip is removably coupled to a distal end of hollow needle 172, as shown in Fig. 2A. For some applications, hollow needle 172 of deployment tool 170 has an outer cross-sectional area which equals between 90% and 110% (e.g., 100%) of the greatest outer cross-sectional area of tip 188, and tip 188 is shaped so as to removably engage the distal end of hollow needle 172, such as shown in Fig. 2A. Alternatively, tip 188 is smaller than the internal diameter of hollow needle 172, allowing tip 188 to be retracted into the needle. Wire 189 may be solid or hollow (i.e., tubular).

For some applications, wire 189 comprises a shape-memory alloy, which is configured to automatically transition to a predetermined shape upon deployment of tissue-coupling portion 130 on the second side of the cardiac tissue wall. For some of these applications, tissue-coupling portion 130 comprises tip 188, while for others of these applications, tissue-coupling portion 130 does not comprise tip 188.

Reference is still made to Figs. 1 and 2A-C. For some applications, expandable tissue anchor 120 further comprises a second sleeve 198, which encloses a portion of wire 189 of tissue-coupling portion 130. Second sleeve 198 serves to increase the contact area between tissue-coupling portion 130 and the second site of the cardiac tissue wall. As a result, second sleeve 198 may generally reduce the likelihood of wire 189 cutting the cardiac tissue wall, particularly if the tissue is diseased. For some applications, second sleeve 198 has an inner diameter of 0.5 mm and an outer diameter of 1.0 mm, and/or may comprise, for example, porous ePTFE or woven PET fiber. The combined profile of sleeve 190 and second sleeve 198 is less than the inner diameter of hollow needle 172.

Reference is now made to Fig. 3, which is a schematic illustration of an expandable tissue anchor 220, in accordance with an application of the present invention. Except as described below, expandable tissue anchor 220 is generally similar to expandable tissue anchor 120, described hereinabove with reference to Figs. 1 and 2A-C, and like reference numerals refer to like parts. Expandable tissue anchor 220 may implement any of the techniques described hereinabove for expandable tissue anchor 120, including, for example, sealing element 174, even though the sealing element is not shown in Fig. 3. For some applications, an anchor system 250 is provided that comprises expandable tissue anchor 220 and tether 152 affixed to flexible elongate tension member 146 such that the tensile force can be applied to expandable tissue anchor 220 via tether 152 and flexible elongate tension member 146.

An anchor head 296 of expandable tissue anchor 220 supports elongate tissue-coupling portion 130 at a proximal end of the tissue-coupling portion. A lateral surface 299 of anchor head 296, typically near a distal end 292 of anchor head 296, is shaped so as to define at least a portion of a distal opening 294 of a passage 291 through expandable tissue anchor 220. For some applications, anchor head 296 comprises one or more collars 297, such as distal and proximal collars 297A and 297B, as shown. Lateral surface 299 may be defined by distal collar 297A, typically near distal end 292 of anchor head 296. Alternatively, anchor head 296 comprises exactly one collar 297, and distal opening 294 is defined by a lateral surface of the exactly one collar (configuration not shown). Further alternatively, anchor head 296 does not comprise any collars 297, and distal opening 294 of passage 291 is defined by a lateral wall of another portion of anchor head 296, such as of a tubular anchor shaft 232 that anchor head 296 comprises (configuration not shown). For some applications, passage 291 includes more than one passage 291, as shown. Passage 291 is typically a channel, but may also be a groove (e.g., a U-shaped groove).

A portion of flexible elongate tension member 146 is slidably disposed through passage 291. Sleeve 190 and passage 291 are sized and shaped such that the size and shape of passage 291 prevent proximal movement of sleeve 190 past passage 291 (e.g., prevent proximal movement of sleeve 190 into distal opening 294). Sleeve 190 and passage 291 thus limit movement of tissue-coupling portion 130 toward distal end 192 of anchor head 196 upon application of the tensile force to flexible elongate tension member 146. This is the case because sleeve 190 has sufficient axial stiffness and too large an outer diameter to pass through passage 291 (e.g., including distal opening 294), and a distal (far) end of sleeve 190 contacts tissue-coupling portion 130 near a junction between flexible elongate tension member 146 and tissue-coupling portion 130, such as shown in Fig. 2C for expandable tissue anchor 120. Sleeve 190, because of its axial stiffness, thus limits compression and deformation of expanded tissue-coupling portion 130 by flexible elongate tension member 146. A portion of the load (tension) applied by flexible elongate tension member 146 brings sleeve 190 into contact with lateral surface 299 of distal collar 297A near or at distal opening 194. Additional load (tension) applied by flexible elongate tension member 146 (a) increases the total load on expanded tissue-coupling portion 130 (without further compressing or deforming expanded tissue-coupling portion 130), (b) optionally is diverted to elastic or plastic deformation of sleeve 190, and (c) pulls on the entire expandable tissue anchor 120.

Locating distal opening 294 of passage 291 at least partially through lateral surface 299 of distal collar 297A may reduce the load on the curved portion of flexible elongate tension member 146 in the vicinity of distal opening 294. In addition, any damage to the proximal end of sleeve 190 is reduced or avoided because the proximal end of sleeve 190 is oriented perpendicularly with distal opening 294 at the point of contact with the distal opening. Further, in this arrangement, flexible elongate tension member 146 is not subject to shear between sleeve 190 and the edge of distal opening 294.

Reference is now made to Fig. 4, which is a schematic illustration of an expandable tissue anchor 320, in accordance with an application of the present invention. Except as described below, expandable tissue anchor 320 is generally similar to expandable tissue anchor 120, described hereinabove with reference to Figs. 1 and 2A-C, and like reference numerals refer to like parts. Expandable tissue anchor 220 may implement any of the techniques described hereinabove for expandable tissue anchor 120. For some applications, an anchor system 350 is provided that comprises expandable tissue anchor 320 and tether 152 affixed to flexible elongate tension member 146 such that the tensile force can be applied to expandable tissue anchor 320 via tether 152 and flexible elongate tension member 146.

In this configuration, a passage 391 through expandable tissue anchor 320 is defined at least in part by tissue-coupling portion 130 of expandable tissue anchor 320. A distal opening 394 of passage 391 may be defined by wire 189 of tissue-coupling portion 130, which, in this configuration, includes at least a portion that is hollow (i.e., tubular), through which a portion of flexible elongate tension member 146 passes. Optionally, passage 391 is also defined by (a) an anchor head 396 of expandable tissue anchor 120 that supports elongate tissue-coupling portion 130 at a proximal end of the tissue-coupling portion. For some applications, anchor head 396 comprises one or more collars 397, such as distal and proximal collars 397A and 397B, as shown, or exactly one collar 397, and passage 391 passes through the one or more collars 397. For some applications, passage 391 includes more than one passage 391, as shown. Passage 391 is typically a channel, but may also be a groove (e.g., a U-shaped groove).

For some applications, techniques and apparatus described in one or more of the following applications are combined with techniques and apparatus described herein: US Patent 8,475,525 to Maisano et al.; US Patent 8,961,596 to Maisano et al.; US Patent 8,961,594 to Maisano et al.; PCT Publication WO 2011/089601; US Patent 9,241,702 to Maisano et al.; US Provisional Application 61/750,427, filed January 9, 2013; US Provisional Application 61/783,224, filed March 14, 2013; US Provisional Application 61/897,491, filed October 30, 2013; US Provisional Application 61/897,509, filed October 30, 2013; US Patent 9,307,980 to Gilmore et al.; PCT Publication WO 2014/108903; PCT Publication WO 2014/141239; US Provisional Application 62/014,397, filed June 19, 2014; PCT Publication WO 2015/063580; US Patent Application Publication 2015/0119936; US Provisional Application 62/086,269, filed December 2, 2014; US Provisional Application 62/131,636, filed March 11, 2015; US Provisional Application 62/167,660, filed May 28, 2015; PCT Publication WO 2015/193728; PCT Publication WO 2016/087934; US Patent Application Publication 2016/0235533; US Patent Application Publication 2016/0242762; PCT Publication WO 2016/189391; US Patent Application Publication 2016/0262741; US Provisional Application 62/376,685, filed August 18, 2016; US Provisional Application 62/456,206, filed February 8, 2017; US Provisional Application 62/456,202, filed February 8, 2017; US Provisional Application 62/465,410, filed March 1, 2017; and US Provisional Application 62/465,400, filed March 1, 2017.

It will be appreciated by persons skilled in the art that the present invention is not limited to what has been particularly shown and described hereinabove. Rather, the scope of the present invention is defined by the appended claims, and may include both combinations and subcombinations of the various features described hereinabove, as well as variations and modifications thereof that are not in the prior art, which would occur to persons skilled in the art upon reading the foregoing description.

## Claims

1. An expandable tissue anchor (120, 220, 320) configured to be delivered to a cardiac chamber using a deployment tool (170), and to be anchored to a cardiac tissue wall at a target site such that a tensile force can be applied to the expandable tissue anchor (120, 220, 320) and thus to the cardiac tissue wall, once the expandable tissue anchor (120, 220, 320) is deployed, so as to move the cardiac tissue wall at the target site relative to adjacent cardiac tissue, the expandable tissue anchor (120, 220, 320) comprising:
an elongate tissue-coupling portion (130) configured to be delivered in an unexpanded generally elongate configuration through the cardiac tissue wall from a first side of the cardiac tissue wall to a second side of the cardiac tissue wall, the tissue-coupling portion (130) further configured to expand on the second side of the cardiac tissue wall;
a flexible elongate tension member (146), which is coupled to a portion of the tissue-coupling portion (130) of the expandable tissue anchor (120, 220, 320) such that the tensile force can be applied to the tissue-coupling portion (130) after it has been expanded, wherein a portion of the flexible elongate tension member (146) is slidably disposed through a passage (191, 291, 391) defined by the expandable tissue anchor (120, 220, 320);
an anchor head (196, 296, 396) that supports the tissue-coupling portion (130) at a proximal end (134) of the tissue-coupling portion (130); and
a sleeve (190) that encloses a portion of the flexible elongate tension member (146) between (a) the tissue-coupling portion (130) of the expandable tissue anchor (120, 220, 320) and (b) a distal opening (194, 294, 394) of the passage (191, 291, 391), with the sleeve (190) and the passage (191, 291, 391) being sized and shaped such that the size and shape of the passage (191, 291, 391) prevent proximal movement of the sleeve (190) past the passage (191, 291, 391) upon application of the tensile force to the flexible elongate tension member (146), thereby limiting compression and deformation of the expanded tissue-coupling portion (130) by the flexible elongate tension member (146),
**characterised in that**:
the sleeve (190) is axially slidable along the flexible elongate tension member (146), with the sleeve (190) and the passage (191, 291) limiting movement of the tissue-coupling portion (130) toward a distal end (192, 292) of the anchor head (196, 296) upon application of the tensile force to the flexible elongate tension member (146), because (a) the sleeve (190) has sufficient axial stiffness and too large an outer diameter to pass through the passage (191, 291), and (b) a distal end (193) of the sleeve (190) contacts the tissue-coupling portion (130) near a junction (195) between the flexible elongate tension member (146) and the tissue-coupling portion (130); and
the sleeve (190) is configured such that the expanded tissue-coupling portion (130) and the sleeve (190) can be drawn tightly against the second side of the cardiac tissue wall at the target site when the tensile force is applied to the tissue-coupling portion (130) after it has been expanded.

2. The expandable tissue anchor (120, 220, 320) according to Claim 1, wherein the expandable tissue anchor (120, 220, 320) is configured such that when the cardiac tissue wall is a myocardial tissue wall, the tissue-coupling portion (130) of the expandable tissue anchor (120, 220, 320) can be advanced into the pericardial cavity between visceral pericardium and parietal pericardium, generally alongside and against the parietal pericardium, without penetrating the parietal pericardium.

3. The expandable tissue anchor (120, 220, 320) according to Claim 1 or Claim 2, wherein the anchor head (196, 296, 396) is shaped so as to define the passage (191, 291, 391).

4. The expandable tissue anchor (220) according to Claim 3, wherein a lateral surface (299) of the anchor head (299) is shaped so as to define at least a portion of the distal opening (294) of the passage (291).

5. The expandable tissue anchor (120, 220, 320) according to Claim 3, wherein the anchor head comprises a collar (197, 297, 397), which is shaped so as to define the distal opening (194, 294, 394) of the passage (191, 291, 391).

6. The expandable tissue anchor (120) according to Claim 5, wherein a distal end of the collar (197) is shaped so as to define the distal opening (194) of the passage (191).

7. The expandable tissue anchor (220) according to Claim 5, wherein a lateral surface (299) of the collar (297) is shaped so as to define at least a portion of the distal opening (294) of the passage (291).

8. The expandable tissue anchor (120, 220, 320) according to Claim 1 or Claim 2, wherein the tissue-coupling portion (130) of the expandable tissue anchor (120, 220, 320), once expanded on the second side of the cardiac tissue wall, assumes a shape generally orthogonal to the anchor head (196, 296, 396).

9. An anchor system (150, 250, 350) comprising the expandable tissue anchor (120, 220, 320) according to Claim 1, wherein the anchor system (150, 250, 350) further comprises a tether (152) affixed to the flexible elongate tension member (146) such that the tensile force can be applied to the expandable tissue anchor (120, 220, 320) via the tether (152) and the flexible elongate tension member (146).

## Patentansprüche

1. Erweiterbarer Gewebeanker (120, 220, 320), der konfiguriert ist, um mithilfe eines Einsatzwerkzeugs (170) in eine Herzkammer bereitgestellt zu werden und an einer Zielstelle an eine Herzgewebewand verankert zu werden, so dass eine Zugkraft auf den erweiterbaren Gewebeanker (120, 220, 320) und somit auf die Herzgewebewand angelegt werden kann, sobald der erweiterbare Gewebeanker (120, 220, 320) eingesetzt ist, um die Herzgewebewand an der Zielstelle relativ zu benachbartem Herzgewebe zu bewegen, wobei der erweiterbare Gewebeanker (120, 220, 320) umfasst:
einen länglichen Gewebekopplungsabschnitt (130), der konfiguriert ist, um in einer nicht erweiterten allgemein länglichen Konfiguration durch die Herzgewebewand von einer ersten Seite der Herzgewebewand zu einer zweiten Seite der Herzgewebewand bereitgestellt zu werden, wobei der Gewebekopplungsabschnitt (130) ferner konfiguriert ist, um sich an der zweiten Seite der Herzgewebewand zu erweitern;
ein flexibles längliches Spannungselement (146), das an einen Abschnitt des Gewebekopplungsabschnitts (130) des erweiterbaren Gewebeankers (120, 220, 320) gekoppelt ist, so dass die Zugkraft auf den Gewebekopplungsabschnitt (130) angelegt werden kann, nachdem er erweitert wurde, wobei ein Abschnitt des flexiblen länglichen Spannungselements (146) gleitbar durch einen durch den erweiterbaren Gewebeanker (120, 220, 320) definierten Durchgang (191, 291, 391) angeordnet ist;
einen Ankerkopf (196, 296, 396), der den Gewebekopplungsabschnitt (130) an einem proximalen Ende (134) des Gewebekopplungsabschnitts (130) unterstützt; und
eine Hülse (190), die einen Abschnitt des flexiblen länglichen Spannungselements (146) zwischen (a) dem Gewebekopplungsabschnitt (130) des erweiterbaren Gewebeankers (120, 220, 320) und (b) einer distalen Öffnung (194, 294, 394) des Durchgangs (191, 291, 391) umschließt, wobei die Hülse (190) und der Durchgang (191, 291, 391) so größenbemessen und geformt sind, dass die Größe und Form des Durchgangs (191, 291, 391) eine proximale Bewegung der Hülse (190) nach Anwenden der Zugkraft auf das flexible längliche Spannungselement (146) am Durchgang (191, 291, 391) vorbei verhindern, wodurch eine Kompression und Deformation des erweiterten Gewebekopplungsabschnitts (130) durch das flexible längliche Spannungselement (146) beschränkt wird, **dadurch gekennzeichnet, dass**:
die Hülse (190) entlang des flexiblen länglichen Spannungselements (146) axial gleitbar ist, wobei die Hülse (190) und der Durchgang (191, 291) eine Bewegung des Gewebekopplungsabschnitts (130) zu einem distalen Ende (191, 292) des Ankerkopfs (196, 296) nach Anwendung der Zugkraft auf das flexible längliche Spannungselement (146) begrenzt, weil (a) die Hülse (190) eine ausreichende axiale Steifigkeit und einen zu großen Außendurchmesser aufweist, um den Durchgang (191, 291) zu passieren, und (b) ein distales Ende (193) der Hülse (190) den Gewebekopplungsabschnitt (130) nahe einer Verbindung (195) zwischen dem flexiblen länglichen Spannungselement (146) und dem Gewebekopplungsabschnitt (130) kontaktiert; und
die Hülse (190) so konfiguriert ist, dass der erweiterte Gewebekopplungsabschnitt (130) und die Hülse (190) eng gegen die zweite Seite der Herzgewebewand an der Zielstelle gezogen werden können, wenn die Zugkraft auf den Gewebekopplungsabschnitt (130) angelegt wird, nachdem er expandiert wurde.

2. Erweiterbarer Gewebeanker (120, 220, 320) nach Anspruch 1, wobei der erweiterbare Gewebeanker (120, 220, 320) so konfiguriert ist, dass wenn die Herzgewebeweand eine Myokardgewebewand ist, der Gewebekopplungsabschnitt (130) des erweiterbaren Gewebeankers (120, 220, 320) in die Perikardhöhle zwischen dem viszeralen Perikard und dem parietalen Perikard, allgemein entlang und gegen den parietalen Perikard, vorgeschoben werden, ohne den parietalen Perikard zu penetrieren.

3. Erweiterbarer Gewebeanker (120, 220, 320) nach Anspruch 1 oder Anspruch 2, wobei der Ankerkopf (196, 296, 396) so geformt ist, um den Durchgang (191, 291, 391) zu definieren.

4. Erweiterbarer Gewebeanker (220) nach Anspruch 3, wobei eine laterale Fläche (299) des Ankerkopfs (299) so geformt ist, um mindestens einen Abschnitt der distalen Öffnung (294) des Durchgangs (291) zu definieren.

5. Erweiterbarer Gewebeanker (120, 220, 320) nach Anspruch 3, wobei der Ankerkopf einen Kragen (197, 297, 397) umfasst, der so geformt ist, um die distale Öffnung (194, 294, 394) des Durchgangs (191, 291, 391) zu definieren.

6. Erweiterbarer Gewebeanker (120) nach Anspruch 5, wobei ein distales Ende des Kragens (197) so geformt ist, um die distale Öffnung (194) des Durchgangs (191) zu definieren.

7. Erweiterbarer Gewebeanker (220) nach Anspruch 5, wobei eine laterale Fläche (299) des Kragens (297) so geformt ist, um mindestens einen Abschnitt der distalen Öffnung (294) des Durchgangs (291) zu definieren.

8. Erweiterbarer Gewebeanker (120, 220, 320) nach Anspruch 1 oder Anspruch 2, wobei der Gewebekopplungsabschnitt (130) des erweiterbaren Gewebeankers (120, 220, 320), nachdem er auf der zweiten Seite der Herzgewebewand erweitert ist, eine zum Ankerkopf (196, 296, 396) allgemein orthogonale Form annimmt.

9. Ankersystem (150, 250, 350), umfassend den erweiterbaren Gewebeanker (120, 220, 320) nach Anspruch 1, wobei das Ankersystem (150, 250, 350) ferner einen Haltegurt (152) umfasst, der am flexiblen länglichen Spannungselement (146) befestigt ist, so dass die Zugkraft an den erweiterbaren Gewebeanker (120, 220, 320) über den Haltegurt (152) und das flexible längliche Spannungselement (146) angelegt werden kann.

## Revendications

1. Ancre de tissu extensible (120, 220, 320) conçue pour être délivrée à une cavité cardiaque à l'aide d'un outil de déploiement (170) et pour être ancrée à une paroi de tissu cardiaque au niveau d'un site cible de telle sorte qu'une force de traction puisse être appliquée sur l'ancre de tissu extensible (120, 220, 320) et par conséquent à la paroi de tissu cardiaque, une fois que l'ancre de tissu extensible (120, 220, 320) est déployée, de manière à déplacer la paroi de tissu cardiaque au niveau du site cible par rapport au tissu cardiaque adjacent, l'ancre de tissu extensible (120, 220, 320) comprenant :
une partie allongée de couplage de tissu (130) configurée pour être délivrée dans une configuration non élargie généralement allongée à travers la paroi de tissu cardiaque d'un premier côté de la paroi de tissu cardiaque à un second côté de la paroi de tissu cardiaque, la partie de couplage de tissu (130) étant en outre conçue pour s'élargir sur le second côté de la paroi de tissu cardiaque ;
un élément de tension allongé flexible (146), qui est couplé à une partie de la partie de couplage de tissu (130) de l'ancre de tissu extensible (120, 220, 320) de sorte que la force de traction puisse être appliquée sur la partie de couplage de tissu (130) après son élargissement, une partie de l'élément de tension allongé flexible (146) étant disposée coulissante à travers un passage (191, 291, 391) défini par l'ancre de tissu extensible (120, 220, 320) ;
une tête d'ancrage (196, 296, 396) qui supporte la partie de couplage de tissu (130) au niveau d'une extrémité proximale (134) de la partie de couplage de tissu (130) ; et
un manchon (190) qui renferme une partie de l'élément de tension allongé flexible (146) entre (a) la partie de couplage de tissu (130) de l'ancre de tissu extensible (120, 220, 320) et (b) une ouverture distale (194, 294, 394) du passage (191, 291, 391), le manchon (190) et le passage (191, 291, 391) étant dimensionnés et façonnés de telle sorte que la taille et la forme du passage (191, 291, 391) empêchent le déplacement proximal du manchon (190) après le passage (191, 291, 391) lors de l'application de la force de traction sur l'élément de tension allongé flexible (146), limitant ainsi la compression et la déformation de la partie de couplage de tissu élargie (130) par l'élément de tension allongé flexible (146),
**caractérisé en ce que** :
le manchon (190) peut coulisser axialement le long de l'élément de tension allongé flexible (146), le manchon (190) et le passage (191, 291) limitant le déplacement de la partie de couplage de tissu (130) vers une extrémité distale (192, 292) de la tête d'ancrage (196, 296) lors de l'application de la force de traction sur l'élément de tension allongé flexible (146), parce que (a) le manchon (190) a une rigidité axiale suffisante et un diamètre externe trop grand pour passer à travers le passage (191, 291) et (b) une extrémité distale (193) du manchon (190) entre en contact avec la partie de couplage de tissu (130) à proximité d'une jonction (195) entre l'élément de tension allongé flexible (146) et la partie de couplage de tissu (130) ; et
le manchon (190) est conçu de telle sorte que la partie de couplage de tissu élargie (130) et le manchon (190) peuvent être tirés étroitement contre le second côté de la paroi de tissu cardiaque au niveau du site cible lorsque la force de traction est appliquée sur la partie de couplage de tissu (130) après son élargissement.

2. Ancre de tissu extensible (120, 220, 320) selon la revendication 1, ladite ancre de tissu extensible (120, 220, 320) étant conçue de telle sorte que lorsque la paroi de tissu cardiaque est une paroi de tissu myocardique, la partie de couplage de tissu (130) de l'ancre de tissu extensible (120, 220, 320) peut être avancée dans la cavité péricardique entre le péricarde viscéral et le péricarde pariétal, généralement le long et contre le péricarde pariétal, sans pénétrer dans le péricarde pariétal.

3. Ancre de tissu extensible (120, 220, 320) selon la revendication 1 ou la revendication 2, ladite tête d'ancrage (196, 296, 396) étant formée de manière à définir le passage (191, 291, 391).

4. Ancre de tissu extensible (220) selon la revendication 3, une surface latérale (299) de la tête d'ancrage (299) étant formée de manière à définir au moins une partie de l'ouverture distale (294) du passage (291).

5. Ancre de tissu extensible (120, 220, 320) selon la revendication 3, ladite tête d'ancrage comprenant un collier (197, 297, 397) qui est formé de manière à définir l'ouverture distale (194, 294, 394) du passage (191, 291, 391).

6. Ancre de tissu extensible (120) selon la revendication 5, une extrémité distale du collier (197) étant formée de manière à définir l'ouverture distale (194) du passage (191).

7. Ancre de tissu extensible (220) selon la revendication 5, une surface latérale (299) du collier (297) étant formée de manière à définir au moins une partie de l'ouverture distale (294) du passage (291).

8. Ancre de tissu extensible (120, 220, 320) selon la revendication 1 ou la revendication 2, ladite partie de couplage de tissu (130) de l'ancre de tissu extensible (120, 220, 320), une fois élargie sur le second côté de la paroi de tissu cardiaque, prenant une forme généralement orthogonale à la tête d'ancrage (196, 296, 396).

9. Système d'ancrage (150, 250, 350) comprenant l'ancre de tissu extensible (120, 220, 320) selon la revendication 1, ledit système d'ancrage (150, 250, 350) comprenant en outre une attache (152) fixée sur l'élément de tension allongé flexible (146) de sorte que la force de traction puisse être appliquée sur l'ancre de tissu extensible (120, 220, 320) via l'attache (152) et l'élément de tension allongé flexible (146).
